Europäisches Patentamt
European Patent Office
Office européen des brevets

(19)

(11) Veröffentlichungsnummer : **0 046 532**
**B1**

(12) **EUROPÄISCHE PATENTSCHRIFT**

(45) Veröffentlichungstag der Patentschrift : **14.09.83**

(21) Anmeldenummer : **81106197.7**

(22) Anmeldetag : **07.08.81**

(51) Int. Cl.³ : **C 07 C 43/23**, C 07 C 49/255, C 07 C 69/76, C 07 D233/60, C 07 D249/08, C 07 C 41/26, C 07 C 45/71// A01N39/00, A01N43/50, A01N43/64, C07C43/295, C07C49/16, C07C79/35, C07C79/36, C07C45/63, C07C76/02

(54) **3,3-Dimethyl-1-fluor-1-phenoxy-2-butanone(ole), Verfahren zu ihrer Herstellung und ihre Verwendung.**

(30) Priorität : **23.08.80 DE 3031846**

(43) Veröffentlichungstag der Anmeldung : **03.03.82 Patentblatt 82/09**

(45) Bekanntmachung des Hinweises auf die Patenterteilung : **14.09.83 Patentblatt 83/37**

(84) Benannte Vertragsstaaten : **BE CH DE FR GB IT LI NL**

(56) Entgegenhaltungen : **DE B 2 201 063**

(73) Patentinhaber : **BAYER AG**
**Zentralbereich Patente, Marken und Lizenzen**
**D-5090 Leverkusen 1, Bayerwerk (DE)**

(72) Erfinder : **Kraatz, Udo, Dr.**
**Körner Strasse 6**
**D-5090 Leverkusen 1 (DE)**
Erfinder : **Stetter, Jörg, Dr.**
**Gellertweg 4**
**D-5600 Wuppertal 1 (DE)**
Erfinder : **Büchel, Karl Heinz, Prof.Dr.**
**Dabringhausener Strasse 42**
**D-5093 Burscheid (DE)**

**EP 0 046 532 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

3,3-Dimethyl-1-fluor-1-phenoxy-2-butanone(ole), Verfahren zu ihrer Herstellung und ihre Verwendung

Die vorliegende Erfindung betrifft neue 3,3-Dimethyl-1-fluor-1-phenoxy-2-butanone(ole), ein Verfahren zu ihrer Herstellung und ihre Verwendung zur Herstellung fungizider Wirkstoffe.

Es ist bereits bekannt geworden, daß bestimmte 3,3-Dimethyl-1-halogen-1-phenoxy-2-butanone, wie z. B. 1-Brom-(Chlor)-3,3-dimethyl-1-phenoxy-2-butanone, interessante Zwischenprodukte zur Herstellung von Pflanzenschutzmitteln sind (vgl. DE-OS 2 201 063 und DE-OS 2 325 156). Die entsprechenden 1-Fluor-Derivate sind bisher noch nicht bekannt.

Es wurden neue, 3,3-Dimethyl-1-fluor-1-phenoxy-2-butanone-(ole) der allgemeinen Formel (I)

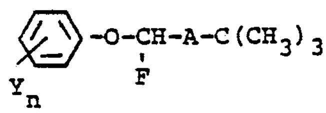

(I)

in welcher

A für die Ketogruppe oder die CH(OH)-Gruppierung steht,

Y für Halogen, für Alkyl und Alkoxy mit jeweils 1 bis 4 Kohlenstoffatomen, Cycloalkyl mit 5 bis 7 Kohlenstoffatomen, für die Nitrogruppe und weiterhin für gegebenenfalls durch Halogen oder Alkyl mit 1 bis 2 Kohlenstoffatomen substituiertes Phenyl und Phenoxy steht, sowie für die Gruppierung —CO—OR steht, wobei

R für Alkyl mit 1 bis 4 Kohlenstoffatomen und gegebenenfalls durch Halogen oder Alkyl mit 1 bis 2 Kohlenstoffatomen substituiertes Phenyl oder Benzyl steht, und

n für ganze Zahlen von 0 bis 3 steht,

gefunden.

Weiterhin wurde gefunden, daß man die neuen 3,3-Dimethyl-1-fluor-1-phenoxy-2-butanone(ole) der Formel (I) erhält, wenn man Halogenketone der Formel (II)

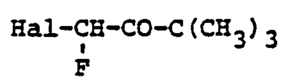

(II)

in welcher

Hal für Chlor oder Brom steht,

mit Phenolen der Formel (III)

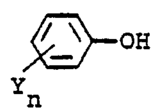

(III)

in welcher

Y und n die oben angegebene Bedeutung haben,

in Gegenwart eines Verdünnungsmittels und in Gegenwart eines Säurebindemittels umsetzt und gegebenenfalls die 5 entstehenden Keto-Derivate der Formel (Ia)

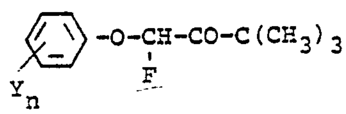

(Ia)

in welcher

Y und n die oben angegebene Bedeutung haben,

in üblicher Weise reduziert.

Die neuen Verbindungen der Formel (I) sind interessante Zwischenprodukte zur Herstellung von

Pflanzenschutz-Wirkstoffen mit fungizider Wirkung.

Sie erlauben eine bessere Herstellung von den aus DE-OS 2 201 063 und DE-OS 2 325 156 bekannten Pflanzenschutzmitteln, die als Substituent Y Reste tragen, die gegen Halogenierungsmittel empfindlich sind (wie Alkenyl, Alkinyl, Alkylthio, Alkylcarbonyl) oder die als Elektronendonatoren den Eintritt von Halogen in den Kern erleichtern (wie Alkoxy, Amino, Mono- und Dialkylamino) als die aus den genannten Offenlegungsschriften bekannten entsprechenden Bromide und Chloride.

Der erfindungsgemäßen 3,3-Dimethyl-1-fluor-1-phenoxy-2-butanone(ole) sind durch die Formel (I) allgemein definiert. In dieser Formel steht Y vorzugsweise für Fluor, Chlor, Brom, Methyl, Ethyl, Methoxy, Methoxycarbonyl, Cyclohexyl, Phenyl, Chlorphenyl, Phenoxy, Chlorphenoxy oder Nitro steht, und A sowie der Index n die in der Erfindungsdefinition angegebene Bedeutung haben.

Im einzelnen seien außer den bei den Herstellungsbeispielen genannten Verbindungen folgende Verbindungen der allgemeinen Formel (I) genannt :

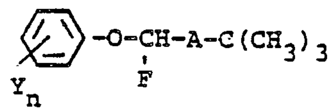

(I)

| $Y_n$ | A |
|---|---|
| 4-F | CO oder CH(OH) |
| 4-Br | CO oder CH(OH) |
| $4-NO_2$ | CO oder CH(OH) |
| $2,4-Cl_2$ | CO oder CH(OH) |
| 3-Cl | CO oder CH(OH) |
| 4-Br, 2-Cl | CO oder CH(OH) |
| $2-OCH_3$ | CO oder CH(OH) |
| $2,4-(CH_3)_2$ | CO oder CH(OH) |
| $3,4-Cl_2$ | CO oder CH(OH) |
| $3-Cl, 4-NO_2$ | CO oder CH(OH) |
| $2-CH_3, 5-NO_2$ | CO oder CH(OH) |
| 2-Br, 4-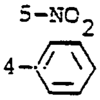 | CO oder CH(OH) |
| 4-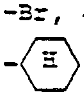 | CO oder CH(OH) |
| 2-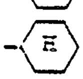 | CO oder CH(OH) |
| 4-O-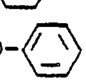 | CO oder CH(OH) |
| $2,6-Cl_2$, 4-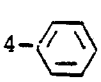 | CO oder CH(OH) |
| $2,4,6-Cl_3$ | CO oder CH(OH) |
| 2-Cl, 4-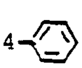 | CO oder CH(OH) |
| $4-C(CH_3)_3$ | CO oder CH(OH) |
| 2-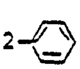 | CO oder CH(OH) |
| $2,6-Cl_2$ | CO oder CH(OH) |
| $2,5-Cl_2$ | CO oder CH(OH) |

(Fortsetzung)

| $Y_n$ | A |
|---|---|
| 2-Cl, 6-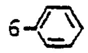 | CO oder CH(OH) |
| 4-CO-$OCH_3$ | CO oder CH(OH) |
| 4-$CH_3$ | CO oder CH(OH) |
| 4-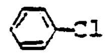 | CO oder CH(OH) |
| 4-Cl, 2-$CH_3$ | CO oder CH(OH) |

Verwendet man beispielsweise 1-Brom-3,3-dimethyl-1-fluor-2-butanon und 4-Chlorphenol als Ausgangsstoffe, so kann der Reaktionsablauf durch das folgende Formelschema wiedergegeben werden :

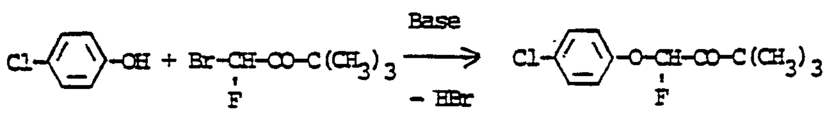


Verwendet man beispielsweise 1-(4-Chlorphenoxy)-3,3-dimethyl-1-fluor-2-butanon und Natriumborhydrid als Ausgangsstoffe, so kann der Reaktionsablauf durch das folgende Formelschema wiedergegeben werden :

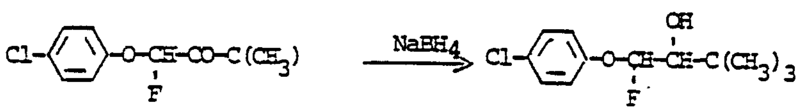


Die bei der Durchführung des erfindungsgemäßen Verfahrens als Ausgangsstoffe benötigten Halogenketone der Formel (II) sind noch nicht bekannt ; sie sind jedoch unter anderem Gegenstand einer eigenen älteren Anmeldung, die noch nicht veröffentlicht ist (vgl. die deutsche Patentanmeldung P 2 937 595 vom 18.9.1979 (Le A 19 934)) ; und werden erhalten, indem man im bekannten 1-Brom(chlor)-3,3-dimethyl-2-butanon der Formel

$$(Cl)Br{-}CH_2{-}CO{-}C(CH_3)_3 \qquad (IV)$$

das Brom bzw. Chlor in üblicher Weise gegen Fluor austauscht und im entstehenden 3,3-Dimethyl-1-fluor-2-butanon der Formel

$$F{-}CH_2{-}CO{-}C(CH_3)_3 \qquad (V)$$

eines der beiden aktiven Wasserstoffatome in üblicher Weise gegen Brom oder Chlor austauscht (vgl. auch die Herstellungsbeispiele).

Die außerdem für das erfindungsgemäße Verfahren als Ausgangsstoffe benötigten Phenole sind durch die Formel (III) allgemein definiert. In dieser Formel stehen Y und der Index n vorzugsweise für diejenigen Bedeutungen, die bereits bei der Beschreibung der erfindungsgemäßen Stoffe der Formel (I) vorzugsweise genannt wurden.

Die Phenole der Formel (III) sind allgemein bekannte Verbindungen der organischen Chemie.

Für die erfindungsgemäße Umsetzung kommen als Verdünnungsmittel vorzugsweise inerte organische Lösungsmittel infrage. Hierzu gehören vorzugsweise Ketone, wie Aceton, Methylethylketon oder Methyl-isobutylketon ; Nitrile, wie Acetonitril oder Propionitril ; Ether, wie Tetrahydrofuran oder Dioxan ; Alkohole, wie Ethanol oder Isopropanol ; aromatische Kohlenwasserstoffe, wie Benzol ; sowie auch Dimethylformamid.

Die erfindungsgemäße Umsetzung wird in Gegenwart eines Säurebinders vorgenommen. Man kann alle üblicherweise verwendbaren anorganischen oder organischen Säurebinder zugeben, wie Alkalimetallcarbonate, beispielsweise Kaliumcarbonat oder Natriumcarbonat, Alkalimetallhydroxide, beispielsweise Natriumhydroxid, Alkalimetallalkoholate, oder wie niedere tertiäre Alkylamine, beispielsweise Triethylamin.

Die Reaktionstemperaturen können bei der Durchführung des erfindungsgemäßen Verfahrens in einem größeren Bereich variiert werden. Im allgemeinen arbeitet man zwischen 0 und 140 °C, vorzugsweise zwischen 20 und 100 °C.

Bei der Durchführung des erfindungsgemäßen Verfahren arbeitet man vorzugsweise in molaren

Mengen ; es ist aber auch möglich, die Phenole der Formel (III) und den Säurebinder im Überschuß einzusetzen. Die Isolierung der Verbindungen der Formel (I) erfolgt in üblicher Weise.

Die erfindungsgemäße Reduktion erfolgt in allgemein üblicher Weise z. B. durch Umsetzung mit komplexen Hydriden, gegebenenfalls in Gegenwart eines Verdünnungsmittels oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel infrage. Hierzu gehören vorzugsweise Alkohole, wie Methanol, Ethanol, Butanol, Isopropanol und Ether, wie Diethylether oder Tetrahydrofuran. Die Reaktion wird im allgemeinen bei 0 bis 30 °C, vorzugsweise bei 0 bis 20 °C durchgeführt. Hierzu setzt man auf 1 Mol des Ketons der Formel (Ia) etwa 1 Mol eines komplexen Hydrids, wie Natriumhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden ; im allgemeinen arbeitet man zwischen 20 und 120 °C, vorzugsweise bei 50 bis 100 °C. Zur Durchführung der Reaktion setzt man auf 1 Mol des Ketons der Formel (Ia) etwa 1 bis 2 Mol Aluminiumisopropylat ein. Zur Isolierung der reduzierten Verbindungen der Formel (I) wird das überschüssige Lösungsmittel durch Destillation im Vakuum entfernt und die entstandene Aluminium-Verbindung mit verdünnter Schwefelsäure oder Natronlauge zersetzt. Die weitere Aufarbeitung erfolgt in üblicher Weise.

Wie schon erwähnt, stellen die neuen 3,3-Dimethyl-1-fluor-1-phenoxy-2-butanone(ole) der Formel (I) interessante Zwischenprodukte dar. Sie lassen sich leicht in 1-Azolyl-3,3-dimethyl-1-phenoxy-2-butanone(ole) der Formel

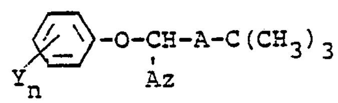

(VI)

in welcher

Az für 1,2,4-Triazol-1-yl, 1,2,4-Triazol-4-yl oder Imidazol-1-yl steht und

A, Y und n die obenangegebene Bedeutung haben,

überführen, indem man die Verbindungen der Formel (I) in der Schmelze bei 100 bis 200 °C mit 1,2,4-Triazol oder Imidazol umsetzt, oder indem man Verbindungen der Formel (I) in üblicher Weise mit Alkalimetallsalzen des 1,2,4-Triazols oder des Imidazols in einem inerten organischen Lösungsmittel, wie z. B. Acetonitril, bei Temperaturen zwischen 20 bis 100 °C umsetzt. Verbindungen der Formel (VI), in denen A für die CH(OH)-Gruppierung steht, können auch erhalten werden, indem man zunächst Verbindungen der Formel (Ia) in oben angegebener Art und Weise zu den 1-Azolyl-3,3-dimethyl-1-phenoxy-2-butanonen der Formel (VI) umsetzt und anschließend die Ketogruppe in üblicher Weise reduziert.

Die 1-Azolyl-3,3-dimethyl-1-phenoxy-2-butanone(ole) der Formel (VI) weisen starke fungizide Eigenschaften auf (vgl. DE-AS 2 201 063, DE-OS 2 324 010, DE-OS 2 325 156 und DE-OS 2 333 354).

In entsprechenden Aufwandmengen bzw. Konzentrationen zeigen auch die erfindungsgemäßen Verbindungen der Formel (I) fungizide Wirksamkeit.

## Herstellungsbeispiele

### Beispiel 1

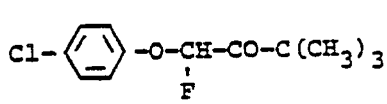

59,1 g (0,3 Mol) 1-Brom-3,3-dimethyl-1-fluor-2-butanon in 50 ml Aceton werden langsam zu 38,5 g (0,3 Mol) 4-Chlorphenol und 42 g (0,3 Mol) Kaliumcarbonat zugetropft, wobei die Temperatur auf 45 °C ansteigt. Man läß eine Stunde weiterrühren. Anschließend wird das anorganische Salz abfiltriert und das Filtrat eingeengt. Der Rückstand wird im Hochvakuum destilliert. Man erhält 63 g (87 % der Theorie) 1-(4-Chlorphenoxy)-3,3-dimethyl-1-fluor-2-butanon vom Siedepunkt 97 °C/0,13 Torr.

Herstellung des Vorproduktes

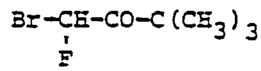

In eine Lösung von 810 g (6,86 Mol) Monofluorpinakolin in 3,5 l Ether werden bei Zimmertemperatur 1,11 kg Brom langsam eingetropft. Die Reaktion ist nur schwach exotherm. Nach der letzten Bromzugabe verschwindet die Bromfarbe rasch. Es wird noch eine Stunde nachgerührt und dann über Nacht stehen gelassen. Nach vorsichtiger Zugabe von 2 000 ml Wasser wird die Etherphase abgetrennt, fünfmal mit je 150 ml Wasser gewaschen und dann über Natriumsulfat getrocknet. Nach Einengen der Etherphase verbleibt eine gelbe Flüssigkeit, die im Wasserstrahlvakuum fraktioniert destilliert wird. Man erhält 1 063, 5 g (78,8 % der Theorie) 1-Brom-3,3-dimethyl-1-fluor-2-butanon vom Siedepunkt 53-57 °C/12 Torr.

Beispiel 2

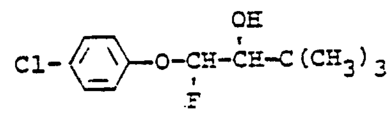

24,2 g (0,1 Mol) 1-(4-Chlorphenoxy)-3,3-dimethyl-1-fluor-2-butanon (Beispiel 1) werden in 200 ml Methanol gelöst und bei Raumtemperatur mit 2 g (50 mMol) Natriumborhydrid versetzt. Man läßt 30 Minuten bei 40 °C nachrühren, engt durch Abdestillieren des Lösungsmittels im Vakuum ein und verteilt den Rückstand zwischen Chloroform/Wasser. Die organische Phase wird abgetrennt, über Natriumsulfat getrocknet und eingeengt. Man erhält nahezu quantitativ 1-(4-Chlorphenoxy)-3,3-dimethyl-1-fluor-2-butanol vom Brechungsindex $n_D^{20}$ : 1,495 9.

Beispiel 3

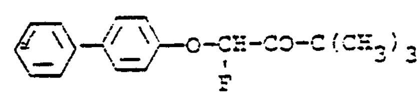

Durch Umsetzung von 1-Brom-3,3-dimethyl-1-fluor-2-butanon mit 4-Phenylphenol entsprechend Beispiel 1 erhält man 1-(4-Biphenylyloxy)-3,3-dimethyl-1-fluor-2-butanon vom Schmelzpunkt 55 °C.

Folgeprodukte

Beispiel a

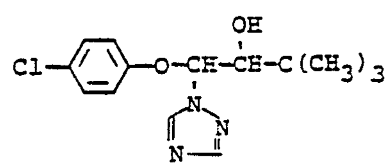

6 g (26 mMol) 1-(4-Chlorphenoxy)-3,3-dimethyl-1-fluor-2-butanol (Beispiel 2) werden mit 5 g (70 mMol) Triazol 3 Stunden auf 130 °C erhitzt. Nach dem Erkalten wird der halbfeste Rückstand zwischen Methylenchlorid/Wasser verteilt, die organische Phase abgetrennt und eingeengt. Das zurückbleibende rohe Endprodukt wird über eine kleine Säule mit Siliciumdioxid chromatographiert. Man erhält in 61 %iger Ausbeute 1-(4-Chlorphenoxy)-3-3dimethyl-1-(1,2,4-triazol-1-yl)-2-butanol vom Schmelzpunkt 114-118 °C.

Beispiel b

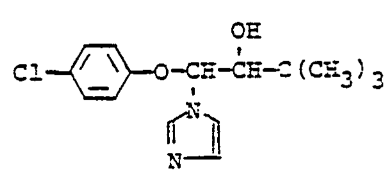

Durch Umsetzung von 1-(4-Chlorphenoxy)-3,3-dimethyl-1-fluor-2-butanol (Beispiel 2) mit Imidazol entsprechend Beispiel a erhält man in 54 %iger Ausbeute 1-(4-Chlorphenoxy)-3,3-dimethyl-1-(imidazol-1-yl)-2-butanol vom Schmelzpunkt 130-140 °C.

Beispiel c

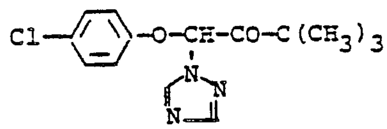

Durch Umsetzung von 1-(4-Chlorphenoxy)-3,3-dimethyl-1-fluor-2-butanon (Beispiel 1) mit 1,2,4-Triazol entsprechend Beispiel a erhält man in 51 %iger Ausbeute 1-(4-Chlorphenoxy)-3,3-dimethyl-1-(1,2,4-triazol-1-yl)-2-butanon vom Schmelzpunkt 74-76 °C.

Beispiel d

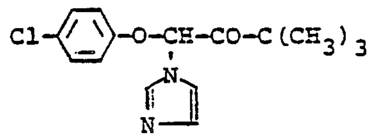

Durch Umsetzung von 1-(4-Chlorphenoxy)-3,3-dimethyl-1-fluor-2-butanon (Beispiel 1) mit Imidazol entsprechend Beispiel a erhält man in 78,5 %iger Ausbeute 1-(4-Chlorphenoxy)-3,3-dimethyl-1-(imidazol-1-yl)-2-butanon vom Schmelzpunkt 94-96 °C.

**Ansprüche**

1. 3,3-Dimethyl-1-fluor-1-phenoxy-2-butanone(ole) der allgemeinen Formel

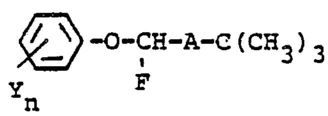

(I)

in welcher

A für die Ketogruppe oder die CH(OH)-Gruppierung steht,

Y für Halogen, für Alkyl und Alkoxy mit jeweils 1 bis 4 Kohlenstoffatomen, Cycloalkyl mit 5 bis 7 Kohlenstoffatomen, für die Nitrogruppe und weiterhin für gegebenenfalls durch Halogen oder Alkyl mit 1 bis 2 Kohlenstoffatomen substituiertes Phenyl und Phenoxy steht, sowie für die Gruppierung —CO—OR steht, wobei

R für Alkyl mit 1 bis 4 Kohlenstoffatomen und gegebenenfalls durch Halogen oder Alkyl mit 1 bis 2 Kohlenstoffatomen substituiertes Phenyl oder Benzyl steht, und

n für ganze Zahlen von 0 bis 3 steht.

2. Verfahren zur Herstellung der 3,3-Dimethyl-1-fluor 1-phenoxy-2-butanone(ole) der Formel (I) in Anspruch 1, dadurch gekennzeichnet, daß man Halogenketone der Formel

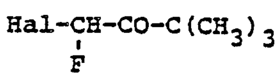

(II)

in welcher

Hal für Chlor oder Brom steht,

mit Phenolen der Formel

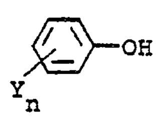

(III)

in welcher

Y und n die in Anspruch 1 angegebene Bedeutung haben,

in Gegenwart eines Verdünnungsmittels und in Gegenwart eines Säurebindemittels umsetzt und gegebenenfalls die entstehenden Keto-Derivate der Formel

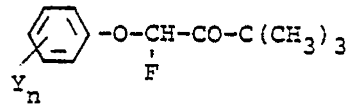

(Ia)

in welcher

Y und n die in Anspruch 1 angegebene Bedeutung haben,

in üblicher Weise reduziert.

3. Verwendung der 3,3-Dimethyl-1-fluor-1-phenoxy-2-butanone(ole) der allgemeinen Formel (I) in Anspruch 1 zur Herstellung der 1-Azolyl-3,3-dimethyl-1-phenoxy-2-butanone(ole) der Formel

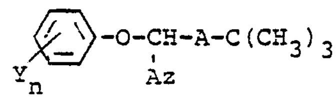

(VI)

in welcher

Az für 1,2,4-Triazol-1-yl, 1,2,4-Triazol-4-yl oder Imidazol-1-yl steht und

A, Y und n die in Anspruch 1 angegebene Bedeutung haben.

**Claims**

1. 3,3-Dimethyl-1-fluoro-1-phenoxy-2-butanones(butanols) of the general formula

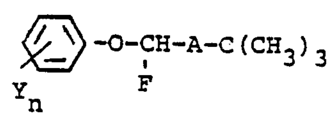

(I)

in which

A represents the keto group or the CH(OH) grouping,

Y represents halogen, alkyl and alkoxy with in each case case 1 to 4 carbon atoms, cycloalkyl with 5 to 7 carbon atoms, the nitro group and furthermore phenoxy and phenyl, optionally substituted by halogen or alkyl with 1 to 2 carbon atoms, as well as the grouping —CO—OR, wherein

R represents alkyl with 1 to 4 carbon atoms and benzyl or phenyl, optionally substituted by halogen or alkyl with 1 to 2 carbon atoms, and

n represents integers from 0 to 3.

2. Process for the preparation of the 3,3-dimethyl-1-fluoro-1-phenoxy-1-butanones(butanols) of the formula (I) in Claim 1, characterised in that halogenoketones of the formula

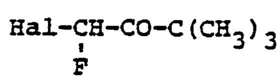

(II)

in which

Hal represents chlorine or bromine,

are reacted with phenols of the formula

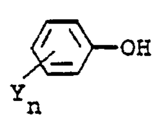

(III)

in which

Y and n have the meaning indicated in Claim 1,

in the presence of a diluent and in the presence of an acidbinding agent and, if appropriate, the keto derivatives formed, of the formula

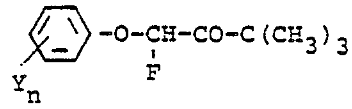

(Ia)

in which

Y and n have the meaning indicated in Claim 1,

are reduced in the customary manner.

3. Use of the 3,3-dimethyl-1-fluoro-1-phenoxy-2-butanones(butanols) of the general formula (I) in Claim 1 for the preparation of the 1-azolyl-3,3-dimethyl-1-phenoxy-2-butanones(butanols) of the formula

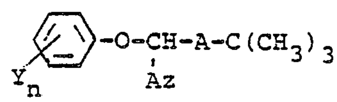

(VI)

in which

Az represents 1, 2, 4-triazol-1-yl, 1, 2, 4-triazol-4-yl or imidazol-1-yl and

A, Y and n have the meaning indicated in Claim 1.

**Revendications**

1. 3,3-diméthyl-1-fluoro-1-phénoxy-2-butanones(ols) de formule générale

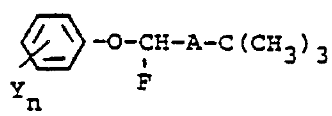

(I)

dans laquelle

A est le groupe céto ou le groupement CH(OH),

Y est un halogène, un groupe alkyle ou alkoxy ayant chacun 1 à 4 atomes de carbone, cycloalkyle ayant 5 à 7 atomes de carbone, le groupe nitro et, en outre, un groupe phényle et un groupe phénoxy éventuellement substitués par un halogène ou un radical alkyle ayant 1 ou 2 atomes de carbone, ainsi que le groupement —CO—OR,

R est un groupe alkyle ayant 1 à 4 atomes de carbone et un groupe phényle ou benzyle éventuellement substitué par un halogène ou un radical alkyle ayant 1 ou 2 atomes de carbone, et

n représente les nombres entiers de 0 à 3.

2. Procédé de production des 3,3-diméthyl-1-fluoro-1-phénoxy-2-butanones(ols) de formule (I) suivant la revendication 1, caractérisé en ce qu'on fait réagir des halogénocétones de formule

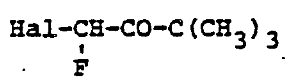

(II)

dans laquelle

Hal représente le chlore ou le brome,

avec des phénols de formule

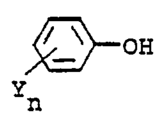

(III)

dans laquelle

Y et n ont la définition indiquée dans la revendication 1,

en présence d'un diluant ou en présence d'un accepteur d'acide et on réduit éventuellement d'une manière classique les dérivés cétoniques produits de formule

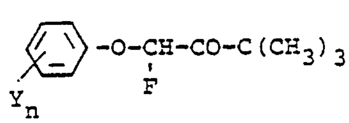

(Ia)

dans laquelle

Y et n ont la définition indiquée dans la revendication 1.

3. Utilisation des 3,3-diméthyl-1-fluoro-1-phénoxy-2-butanones(ols) de formule générale (I) suivant la revendication 1 pour la production des 1-azolyl-3,3-diméthyl-1-phénoxy-2-butanones(ols) de formule

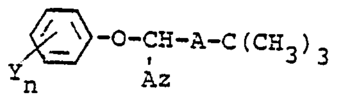 (VI)

dans laquelle

Az est le groupe 1,2,4-triazole-1-yle, 1,2,4-triazole-4-yle ou imidazole-1-yle et

A, Y et n ont la définition indiquée dans la revendication 1.